(19) Europäisches Patentamt
European Patent Office
Office européen des brevets

(11) **EP 2 716 221 A1**

(12) **EUROPEAN PATENT APPLICATION**
published in accordance with Art. 153(4) EPC

(43) Date of publication:
**09.04.2014 Bulletin 2014/15**

(51) Int Cl.:
***A61B 6/00*** *(2006.01)*

(21) Application number: **12791986.8**

(86) International application number:
**PCT/JP2012/003464**

(22) Date of filing: **28.05.2012**

(87) International publication number:
**WO 2012/164901 (06.12.2012 Gazette 2012/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2011   JP 2011120439**
**30.05.2011   JP 2011120440**

(71) Applicant: **FUJIFILM Corporation**
**Tokyo 106-8620 (JP)**

(72) Inventors:
• **NAKATSUGAWA, Haruyasu**
**Ashigarakami-gun**
**Kanagawa 258-8538 (JP)**

• **OHTA, Yasunori**
**Ashigarakami-gun**
**Kanagawa 258-8538 (JP)**
• **NISHINO, Naoyuki**
**Ashigarakami-gun**
**Kanagawa 258-8538 (JP)**
• **YOSHIDA, Yutaka**
**Ashigarakami-gun**
**Kanagawa 258-8538 (JP)**
• **KAMIYA, Takeshi**
**Ashigarakami-gun**
**Kanagawa 258-8538 (JP)**

(74) Representative: **Klunker . Schmitt-Nilson . Hirsch**
**Patentanwälte**
**Destouchesstrasse 68**
**80796 München (DE)**

(54) **METHOD AND DEVICE FOR OBTAINING RADIATION DOSE, AND RADIOGRAPHIC IMAGE PICKUP SYSTEM**

(57)    [Problems to be Solved]

In the case where radiological imaging of a human body with an embedded artificial object is performed, obtaining a more accurate radiation exposure dose of the human body.

[Means for Solving the Problems]

In the case where a radiation exposure dose of a human body that includes an artificial object is obtained based on a radiation image signal detected by a radiation image detector through the application of radiation transmitted through the human body, information of the artificial object included in the human body is obtained and, in the case where information indicating that an artificial object is included in the human body is obtained as the information of the artificial object, a correction is performed to increase the radiation exposure dose, which is based on the radiation image signal, by a predetermined correction radiation exposure dose.

**FIG.1**

## Description

Technical Field

The present invention relates to a radiation exposure dose obtaining method and apparatus, and a radiation image capturing system that obtain, based on a radiation image signal obtained through radiological imaging of a human body that includes an artificial object, a radiation exposure dose of the human body.

Background Art

Recently, it has been regarded as important to manage exposure doses of a patient due to radiological imaging in the medical front where radiological imaging is performed. As the relationship between the exposure dose of the patient due to radiological imaging and a disease such as cancer is drawing attention, it is necessary to maintain the exposure dose of a patient due to radiological imaging and to understand and manage the radiation exposure dose cumulatively received by the patient.

Further, in the medical front where radiological imaging is performed, radiological imaging of moving pictures, such as fluoroscopic images, may be performed, as well as such radiological imaging of still images of patients. In the case of radiological imaging of such moving pictures, a large number of radiation images will be captured at a predetermined frame rate and the radiation exposure dose of the patient will be greater than that in the case where a still image is captured.

Consequently, the radiation exposure dose management of patients in radiological imaging of such moving pictures becomes more important.

As for the method for measuring a radiation expose dose received by a patient by the radiological imaging, for example, a method in which an area dosimeter is provided in a radiation source that emits radiation and the radiation exposure dose received by the patient is obtained based on the value measured by the dosimeter is proposed.

The employment of such method, however, requires a new dosimeter for measuring the exposure dose of the patient, causing a problem of cost increase.

Consequently, instead of providing such dosimeter described above, it is proposed that the exposure dose of a patient is obtained based on an image signal detected by a radiation image detector that detects a radiation image of the patient (refer to, for example, Patent Document 1).

[Prior Art Documents]

[Patent Documents]

Patent Document 1: Japanese Patent No. 4387644

Disclosure of the Invention

In the medical front where radiological imaging is performed, however, there may be a case in which radiological imaging is performed for a patient with an artificial object, such as an artificial bone, embedded in the body. In such a case, if the exposure dose of the patient is tried to be obtained based simply on the image signal of the radiation image detector as in Patent Document 1, there arises a concern that the calculated value may possibly differ from the actual exposure dose of the patient. That is, the radiation is absorbed by the artificial object and the amount of radiation reached the radiation image detector is reduced.

Further, Patent Document 1 proposes that an exposure dose of a patient is obtained in the radiological imaging of a general still image, but proposes nothing about acquisition of a radiation exposure dose in the capturing of such fluoroscopic images described above.

As for the method of capturing fluoroscopic images, for example, there may be a method in which image capturing is performed by moving the radiation application area relative to the patient, but not all of the frames in the fluoroscopic image imaging but some of them will include an image of the artificial object. Therefore, it is necessary to obtain the exposure dose of the patient throughout the fluoroscopic image capturing by taking into account the point described above.

In view of the circumstances described above, it is an object of the present invention to provide a radiation exposure dose obtaining method and apparatus, and a radiation image capturing system capable of obtaining a more accurate radiation exposure dose of a human body even in the case where radiological imaging of the human body is performed.

It is a further object of the present invention to provide a radiation exposure dose obtaining method and apparatus capable of obtaining a more accurate radiation exposure dose of a human body embedded with an artificial object even in the case where radiological imaging of the human body is performed continuously by moving the radiation application range relative to the human body.

A radiation exposure dose obtaining apparatus of the present invention includes an artificial object information obtaining unit that obtains information of an artificial object included in a human body which is an imaging target of radiological imaging and a radiation exposure dose obtaining unit that obtains a radiation exposure dose of the human body based on a radiation image signal detected by a radiation image detector through the application of radiation transmitted through the human body, wherein, in the case where information indicating that an artificial object is included in the human body is obtained by the artificial object information obtaining unit, the radiation exposure dose obtaining unit performs a correction to increase the radiation exposure dose, which is based on the radiation image signal, by a predetermined correction radiation exposure dose.

In the radiation exposure dose obtaining apparatus of the present invention described above, a correspondence relationship between identification information of the human body and information of the artificial object of the human body may be preset, and the artificial object information obtaining unit may be configured to receive identification information of the human body and to obtain information of the artificial object of the human body corresponding to the received identification information of the human body.

Further, the identification information of the human body may be included in an imaging menu of the radiological imaging.

Still further, the artificial object information obtaining unit may be configured to obtain artificial object identification information for identifying the artificial object as the information of the artificial object, and the radiation exposure dose obtaining unit may be configured to include a table in which the artificial object identification information and the correction radiation dose are related, and to perform the correction based on the artificial object identification information obtained by the artificial object information obtaining unit and the table.

Further, the radiation exposure dose obtaining unit may be configured to obtain the radiation exposure dose of the human body based on a radiation image signal of each frame detected by the radiation image detector through a continuous application of the radiation to the human body and to perform a correction to increase by a correction radiation exposure dose according to the frame rate of the radiation image signal.

Still further, the radiation exposure dose obtaining unit may be configured to obtain the radiation exposure dose of the human body based on a radiation image signal of each frame detected by the radiation image detector through a continuous application of the radiation to the human body while moving the application range of the radiation with respect to the human body and to perform a correction to increase by a correction radiation exposure dose according to the movement speed of the application range of the radiation.

A radiation image capturing system of the present invention includes the radiation exposure dose obtaining apparatus described above and a radiation image capturing apparatus that obtains the radiation image signal by performing the radiological imaging, wherein the radiation image capturing apparatus performs the radiological imaging based on the imaging menu that includes the identification information of the human body.

A radiation exposure dose obtaining method of the present invention obtains, based on a radiation image signal detected by a radiation image detector through the application of radiation transmitted through a human body that includes an artificial object, a radiation exposure dose of the human body, the method including the steps of: obtaining information of the artificial object included in the human body; and performing, in the case where information indicating that an artificial object is included in the human body is obtained as the information of the artificial object, a correction to increase the radiation exposure dose, which is based on the radiation image signal, by a predetermined correction radiation exposure dose.

A radiation exposure dose obtaining apparatus of the present invention includes: a radiation exposure dose obtaining unit that obtains, based on a radiation image signal of each frame detected by a radiation image detector through continuous radiological imaging of a living body that includes an artificial object while moving the application range of the radiation relative to the living body, a radiation exposure dose of the living body due to the continuous radiological imaging; and an artificial object frame identification unit that identifies a frame that includes an image signal representing an image of the artificial object as an artificial object frame from a plurality of frames, wherein the radiation exposure dose obtaining unit corrects the radiation exposure dose based on information of the artificial object frame to obtain a radiation exposure dose of the living body for the radiological imaging of the artificial object frame.

In the radiation exposure dose obtaining apparatus described above, the artificial object frame identification unit may be configured to identify the artificial object frame based on dose information of radiation detected by a radiation dose detection unit provided between the living body and the radiation image detector.

Further, the artificial object frame identification unit may be configured to obtain a temporal variation in the dose information of radiation while the continuous radiological imaging is performed.

Still further, the radiation exposure dose obtaining unit may be configured to correct the radiation exposure dose obtained based on the radiation image signal of the artificial object frame using the dose information of radiation detected by the radiation dose detection unit to obtain a radiation exposure dose of the living body for the radiological imaging of the artificial object frame.

Further, the radiation exposure dose obtaining unit may be configured to correct the radiation exposure dose obtained based on the radiation image signal of the artificial object frame using the temporal variation in the dose information of radiation, the frame rate of the radiological imaging, and the speed of the movement.

Still further, the radiation dose detection unit may be provided on the radiation image detector.

Further, the radiation dose detection unit may be provided at a peripheral portion of the radiation image detector.

Still further, the radiation dose detection unit may be provided on each of at least two opposite sides of the radiation image detector in the direction of the movement.

Further, the artificial object may be provided with an index indicating as being an artificial object and the artificial object identification unit may be configured to identify the artificial object by recognizing an image signal of the index included in the radiation image signal of each frame.

Still further, the index may include information used for the correction of the radiation exposure dose and the radiation exposure dose obtaining unit may be configured to perform the correction of the radiation exposure dose based on the information included in the index.

A radiation exposure dose obtaining method of the present invention obtains, based on a radiation image signal of each frame detected by a radiation image detector through continuous radiological imaging of a living body that includes an artificial object while moving the application range of the radiation relative to the living body, a radiation exposure dose of the living body due to the continuous radiological imaging, the method including the steps of: identifying a frame that includes an image signal representing an image of the artificial object as an artificial obj ect frame from a plurality of frames; and correcting the radiation exposure dose based on information of the identified artificial object frame and obtaining a radiation exposure dose of the living body for the radiological imaging of the artificial object frame.

According to the radiation exposure dose obtaining method and apparatus, and the radiation image capturing system of the present invention, in the case where a radiation exposure dose of a human body that includes an artificial object is obtained based on a radiation image signal detected by a radiation image detector through the application of radiation transmitted through the human body, information of the artificial object included in the human body is obtained and, in the case where information indicating that an artificial object is included in the human body is obtained as the information of the artificial object, a correction is performed to increase the radiation exposure dose, which is based on the radiation image signal, by a predetermined correction radiation exposure dose. This allows a more accurate radiation exposure dose that takes into account the radiation absorption by the artificial object to be obtained.

Further, in the radiation exposure dose obtaining method and apparatus, and the radiation image capturing system of the present invention described above, if an arrangement is adopted in which a correspondence relationship between identification information of the human body and information of the artificial object of the human body is preset and information of the artificial object of the human body corresponding to the received identification information of the human body is obtained, patient information or the like which is already inputted and set may be used as the identification information of the human body, so that the information of the artificial object may be obtained by a simple modification of the existing apparatus.

Further, if a table is provided in which the artificial object identification information and the correction radiation dose are related and the correction is performed based on the received artificial object identification information and the table, a correction corresponding to each of a plurality of artificial objects having different radiation absorption levels may be performed.

In the case where the radiation exposure dose of the human body is obtained based on a radiation image signal of each frame detected by the radiation image detector through a continuous application of the radiation to the human body, a correction to increase by a correction radiation exposure dose is performed according to the frame rate of the radiation image signal, an appropriate correction of the radiation exposure dose according to the number of frames may be performed.

In the case where the radiation exposure dose of the human body is obtained based on a radiation image signal of each frame detected by the radiation image detector through a continuous application of the radiation to the human body while moving the application range of the radiation with respect to the human body, if a correction to increase by a correction radiation exposure dose according to the movement speed of the application range of the radiation is performed, an appropriate correction of the radiation exposure dose according to the number of frames may be performed, as in the above.

According to the radiation exposure dose obtaining method and apparatus of the present invention, in the case where radiological imaging of a living body that includes an artificial object is performed while moving the application range of the radiation relative to the living body and a radiation exposure dose is obtained based on the radiation image signal of each frame obtained by the imaging, a frame that includes an image signal representing an image of the artificial object is identified as an artificial object frame from a plurality of frames based on dose information of radiation detected by a radiation dose detection unit provided, for example, between the living body and the radiation image detector and the radiation exposure dose is corrected based on information of the identified artificial object frame to obtain a radiation exposure dose of the living body for the radiological imaging of the artificial object frame. This allows appropriate identification of an artificial object frame to be made by a simple structure.

In the radiation exposure dose obtaining method and apparatus of the present invention described above, if the radiation exposure dose obtained based on the radiation image signal of the artificial object frame is corrected using the dose information of radiation detected by the radiation dose detection unit to obtain a radiation exposure dose for the radiological imaging of the artificial object frame, an accurate radiation exposure dose may be obtained by a simpler correction method.

Further, if an arrangement is adopted in which an artificial object is provided with an index indicating as being an artificial object, and the artificial object is identified by recognizing an image signal of the index included in the radiation image signal of each frame, the artificial object frame may be identified only by performing the image recognition.

Still further, if an arrangement is adopted in which the index of the artificial obj ect includes information used for the correction of the radiation exposure dose and a correction of the radiation exposure dose is performed based on the information included in the index, the correction may be performed easier without requiring, in particular, complicated calculations.

Brief Description of Drawings

Figure 1 is a block diagram of a radiation image capturing system that uses a first embodiment of the radiation exposure dose obtaining apparatus of the present invention, illustrating an overall schematic configuration thereof.

Figure 2 illustrates a schematic structure of the radiation image capturing apparatus of the first embodiment.

Figure 3 is a flowchart illustrating an operation of the radiation image capturing system that uses the first embodiment of the radiation exposure dose obtaining apparatus of the present invention.

Figure 4 is a timing chart illustrating the relationship between radiation application timing and charge accumulation timing in the radiation image detector.

Figure 5 illustrates an example table in which patient information and artificial object information are related.

Figure 6 illustrates an example table in which the artificial object information and correction radiation exposure dose are related.

Figure 7 illustrates an example table in which the artificial object information, the moving speed of the radiation application unit and the radiation image detector, and the correction radiation exposure dose are related.

Figure 8 illustrates an example table in which the artificial object information, the frame rate of radiological imaging, and the correction radiation exposure dose are related.

Figure 9 is a block diagram of a radiation image capturing system that uses a second embodiment of the radiation exposure dose obtaining apparatus of the present invention, illustrating an overall schematic configuration thereof.

Figure 10 illustrates a schematic structure of the radiation image capturing apparatus of the second embodiment.

Figure 11 illustrates a radiation image detector provided with first and second dose measurement sensors.

Figure 12 is a flowchart illustrating an operation of the radiation image capturing system that uses the second embodiment of the radiation exposure dose obtaining apparatus of the present invention.

Figure 13 illustrates, by way of example, a temporal variation in radiation doses detected by the first and second dose measurement sensors.

Figure 14 illustrates a method of calculating a correction radiation exposure dose in the case where an artificial object frame is captured with the artificial object being protruded from the first and second dose measurement sensors.

Figure 15 illustrates a method of identifying an artificial obj ect frame in the case where the end of the artificial obj ect frame cannot be detected.

Figure 16 illustrates a method of obtaining a radiation exposure dose when an artificial object frame is captured.

Best Mode for Carrying Out the Invention

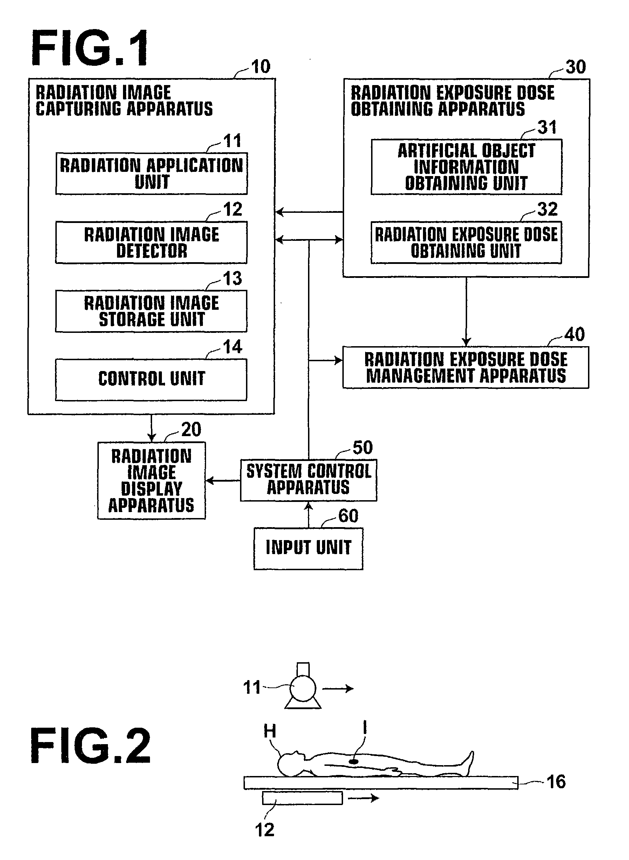

Hereinafter, a radiation image capturing system that uses a first embodiment of the radiation exposure dose obtaining apparatus of the present invention will be described with reference to the accompanying drawings. Figure 1 is a block diagram of the radiation image capturing system of the present embodiment, illustrating an overall schematic configuration thereof.

As illustrated in Figure 1, the radiation image capturing system of the present embodiment includes a radiation image capturing apparatus 10 that captures a fluoroscopic image (moving picture) of a patient while moving the application range of radiation and the radiation image detector relative to the patient, a radiation image display apparatus 20 that displays the fluoroscopic image captured by the radiation image capturing apparatus 10, a radiation exposure dose obtaining apparatus 30 that obtains an exposure dose of the patient based on an image signal of the fluoroscopic image captured by the radiation image capturing apparatus 10, a radiation exposure dose management apparatus 40 that stores and manages the exposure dose of each patient obtained by the radiation exposure dose obtaining apparatus 30, and a system control apparatus 50 that performs overall control of the radiation image capturing system.

As illustrated in Figure 1, the radiation image capturing apparatus 10 includes a radiation application unit 11 that has an X-ray tube, an aperture stop, and the like and applies radiation emitted from the x-ray tube and passed through the aperture stop to a patient, a radiation image detector 12 that detects radiation transmitted through the patient and outputs a radiation image signal representing a radiation image of the patient, a radiation image storage unit 13 that stores the radiation image signal outputted from the radiation image detector 12, and a control unit 14 that performs overall control of the radiation image capturing apparatus 10.

More specifically, the radiation image capturing apparatus 10 of the present embodiment is structured as illustrated in Figure 2 and performs imaging of a fluoroscopic image by placing a patient H with an artificial object I (e.g., artificial bone) embedded in the body on the image capturing platform 16 and moving the radiation application unit 11 and the radiation image detector 12 relative to the patient in the arrow direction of Figure 2.

The radiation image detector 12 allows repeated use for recording and reading of radiation images, and a so-called direct type radiation image detector that records a radiation image by generating and accumulating a charge by directly receiving radiation or a so-called indirect type radiation image detector that records a radiation image by converting radiation first to visible light and then converting the visible light to a charge and accumulating the charge may be used. As for the radiation image signal reading method for reading out the radiation image recorded by accumulating charges in the manner described above, a so-called TFT (thin film transistor) reading method in which a radiation image signal is read by ON/OFF switching thin film transistors and a so-called optical reading method in which a radiation image signal is read out by applying reading light are preferably used, but others may also be used.

The radiation image capturing apparatus 10 is used to capture a fluoroscopic image (moving picture) of a subject with an embedded artificial object as described above, and the control unit 14 controls the radiation application unit 11 and the radiation image detector 12 such that the fluoroscopic image is captured. More specifically, the control unit 14 controls the radiation application unit 11 to apply radiation at a predetermined frame rate and the radiation image detector 12 to perform recording and reading of a radiation image by the application of the radiation. Then, the radiation image signal of each frame outputted from the radiation image detector 12 is sequentially stored in the radiation image storage unit 13.

As for the structure of the radiation image capturing apparatus 10, a structure in which image capturing is performed with the patient being in the lateral position as illustrated in Figure 2 or a structure in which image capturing is performed with the patient being in upright position may be employed. Further, a structure that allows image capturing with the patient being both in upright position and lateral position may be employed.

The radiation image display apparatus 20 generates a display control signal by performing predetermined processing on the radiation image signal of each frame read out from the radiation image storage unit 13 of the radiation image capturing apparatus 10 and displays a fluoroscopic image of the patient on the monitor based on the display control signal.

The radiation exposure dose obtaining apparatus 30 includes an artificial object information obtaining unit 31 that obtains information of an artificial object in the body of a patient (hereinafter, referred to as "artificial object information") and a radiation exposure dose obtaining unit 32 that obtains a radiation exposure dose of the patient based on the radiation image signal of each frame read out from the radiation image storage unit 13 of the radiation image capturing apparatus 10.

The artificial object information obtaining unit 31 includes a table in which patient information and artificial object information are related, and receives patient information included in an imaging menu inputted at an input unit 60, to be described later, then obtains artificial object information corresponding to the received patient information with reference to the aforementioned table, and outputs the obtained artificial object information to the radiation exposure dose obtaining unit 32.

The radiation exposure dose obtaining unit 32 calculates a radiation dose of the patient based on the radiation image signal of each frame as described above and further performs a correction to increase the radiation exposure dose calculated based on the radiation image signal by a predetermined correction radiation exposure dose in the case where artificial object information indicating that an artificial object is embedded is obtained from the artificial object information obtaining unit 31.

Operations of the artificial object information obtaining unit 31 and the radiation exposure dose obtaining unit 32 will be described later in detail.

The radiation exposure dose management apparatus 40 stores the radiation exposure dose obtained by the radiation exposure dose obtaining apparatus 30 and manages the radiation exposure dose of each patient.

The system control apparatus 50 outputs control signals to the radiation image capturing apparatus 10, the radiation image display apparatus 20, the radiation exposure dose obtaining apparatus 30, and the radiation exposure dose management apparatus 40 to control the operations thereof and at the same time performs input/output signal control between these apparatuses.

The system control apparatus 50 is provided with an input unit 60. The input unit 60 receives an input of imaging menu that includes patient information. The patient information includes at least the information for identifying the patient, such as the name and gender of the patient, the patient ID number and the like. The imaging menu may include, for example, imaging region information, a tube voltage, tube current, application time for applying appropriate dose to the imaging region, imaging frame rate of the fluoroscopic image, movement speed of the radiation application unit 11 and the radiation image detector 12, other than the patient information.

The input information received at the input unit 60 is outputted to each apparatus by the system control apparatus 50 as required.

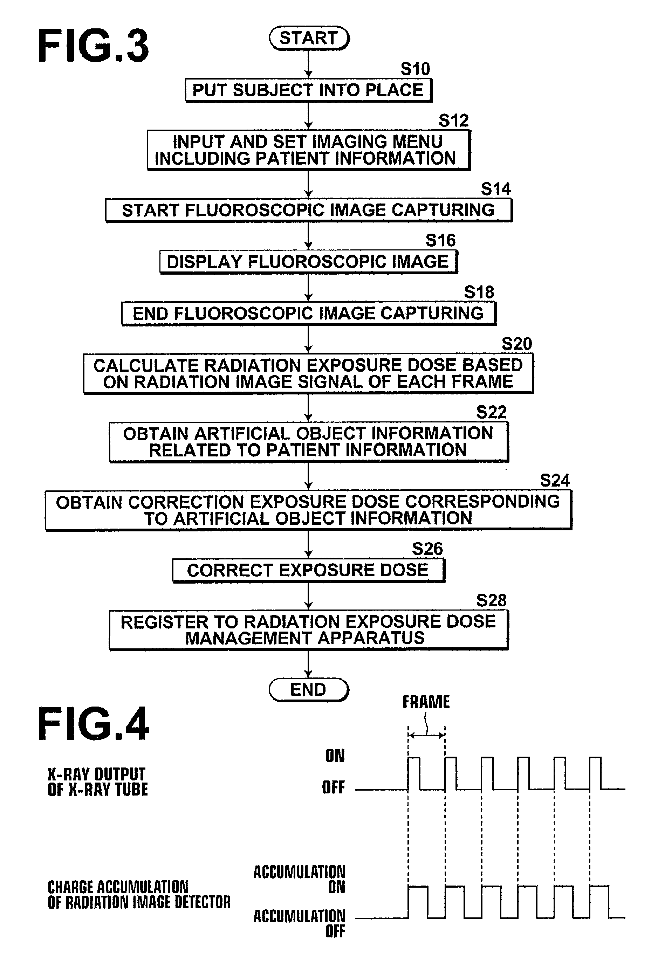

An operation of the radiation image capturing system of the present embodiment will now be described with reference to the flowchart of Figure 3.

First, a patient is placed on an image capturing platform 16 provided in the radiation image capturing apparatus 10 and the patient is put into position (S10).

Then, an imaging menu that includes patient information of the imaging target is inputted by the user using the input unit 60. The patient information included in the imaging menu received at the input unit 60 is outputted to the artificial object information obtaining unit 31 of the radiation exposure dose obtaining apparatus 30 by the system control apparatus 50, and is also outputted to the radiation exposure dose management apparatus 40 and registered therein. The imaging condition included in the imaging menu is outputted to the control unit 14 of the radiation image capturing apparatus 10 and set therein (S12).

Next, an instruction to start fluoroscopic image capturing of the patient is inputted by the user using the input unit 60 and in response to the input, a control signal is outputted from the system control apparatus 50 to the radiation image capturing apparatus 10 to capture a fluoroscopic image, and the radiation image capturing apparatus 10 starts fluoroscopic image capturing according to the inputted control signal (S14).

More specifically, the radiation application unit 11 and the radiation image detector 12 are moved relative to the patient according to the inputted control signal and the X-ray tube of the radiation application unit 11 is controlled based on the inputted imaging condition and a predetermined dose of radiation is applied toward the patient intermittently at a predetermined frame rate.

Then, radiation transmitted through the patient is incident on the radiation image detector 12 and subjected to a photoelectrical conversion in the radiation image detector 12 and accumulated therein as a charge signal.

Thereafter, each time the application of radiation for each frame is ended, the charge signal accumulated in the radiation image detector 12 is read out by the control unit 14, then converted to a digital signal by an A/D converter (not shown), and stored in the radiation image storage unit 13.

Figure 4 is a timing chart illustrating the application timing of radiation from the X-ray tube of the radiation application unit 11 and the charge accumulation timing of the radiation image detector 12. The period during which no charge accumulation is performed in the radiation image detector 12 (accumulation OFF period) is a period for reading out a charge signal from the radiation image detector 12.

The repeated performance of radiation application by the X-ray tube and the radiation image recording and reading in the radiation image detector 12 at a predetermined frame rate in the manner described above causes the radiation image signal of each frame to be sequentially stored in the radiation image storage unit 13.

Then, the radiation image signal of each frame stored in the radiation image storage apparatus 13 is sequentially read out and outputted to the radiation image display apparatus 20. The radiation image display apparatus 20 sequentially generates a display control signal based on the inputted radiation image signal of each frame and sequentially outputs the display control signal to the monitor to display a fluoroscopic image of the patient as a moving picture (S16).

Thereafter, when an instruction to end the fluoroscopic image capturing is inputted by the user using the input unit 60, the system control apparatus 50 outputs a control signal to the radiation image capturing apparatus 10 to end the fluoroscopic image capturing, and the radiation image capturing apparatus 10 ends the fluoroscopic image capturing in response to the inputted control signal (S18).

Next, when the fluoroscopic image capturing described above is ended, a radiation exposure dose of the patient is obtained in the radiation exposure dose obtaining apparatus 30.

More specifically, a radiation exposure dose received by the patient during imaging of each frame of the fluoroscopic image is calculated based on the radiation image signal of each frame in the radiation exposure dose obtaining unit 32 (S20). More specifically, an E.I. (Exposure Index) is calculated first based on the radiation image signal of each frame and the radiation exposure dose is obtained based on the E.I. in the present embodiment.

The E.I. is calculated in the following manner. First, a predetermined calculation area is set in a radiation image of each frame. The calculation area may be, for example, the entire area of the radiation image, an area arbitrarily set by the user, an area defined based on the imaged region information, or an area within 10% of the image size from the center of the radiation image. Otherwise, an area except for the so-called direct exposure area which may be obtained based on, for example, a histogram of the radiation image or an area of 90% of the entire density width from the central density of the radiation image may be employed. Alternatively, a specific calculation area may be set by combining the aforementioned conditions.

Next, a representative value V of the calculation area set in the above is calculated. As for the representative value V, the density value itself of the radiation image or a statistical characteristic value obtained by modifying the density value itself with the average value of the entire density values, median value, mode value, or trimmed mean value may be employed. Then, based on the representative value V, the E.I. of each frame is calculated by the formula given below:

$$E.I. = C_0 \times g(V),$$

where:

g(V): reverse calibration function; and
Co: 100·Gy (constant).

The g(V) is a function defined based on a radiation image obtained with the radiation quality of RQA5. The representative value V differs in magnitude due to difference in sensitivity arising from difference in scintillator used in the radiation image detector or difference in the setting method of the calculation area or the calculation method of the representative value V and g(V) is a function to normalize the differences. That is, if RQA5 radiation is received by any type of radiation image detector by the same radiation dose, the E.I. will become nearly the same value.

The radiation exposure dose received by the patient during imaging of each frame is calculated by the radiation exposure dose obtaining unit 42 using the E.I. calculated in the manner described above. As for the method of obtaining the radiation exposure dose using the E.I., for example, a function which defines these relationships or a lookup table may be set in advance.

Here, if an artificial object is included in the body of the patient who is the imaging target of the fluoroscopic image described above, the radiation applied to the patient is absorbed by the contrast agent before reaching the radiation image detector 12. Therefore, if a radiation exposure dose is calculated based on the radiation image signal which includes an image signal of the artificial object, the calculated value will become smaller than that actually received by the patient.

Consequently, if an artificial object is included in the body of a patient who is the imaging target, a correction to add a correction radiation exposure dose to the radiation exposure dose calculated based on the radiation image signal as described above is performed in the present embodiment.

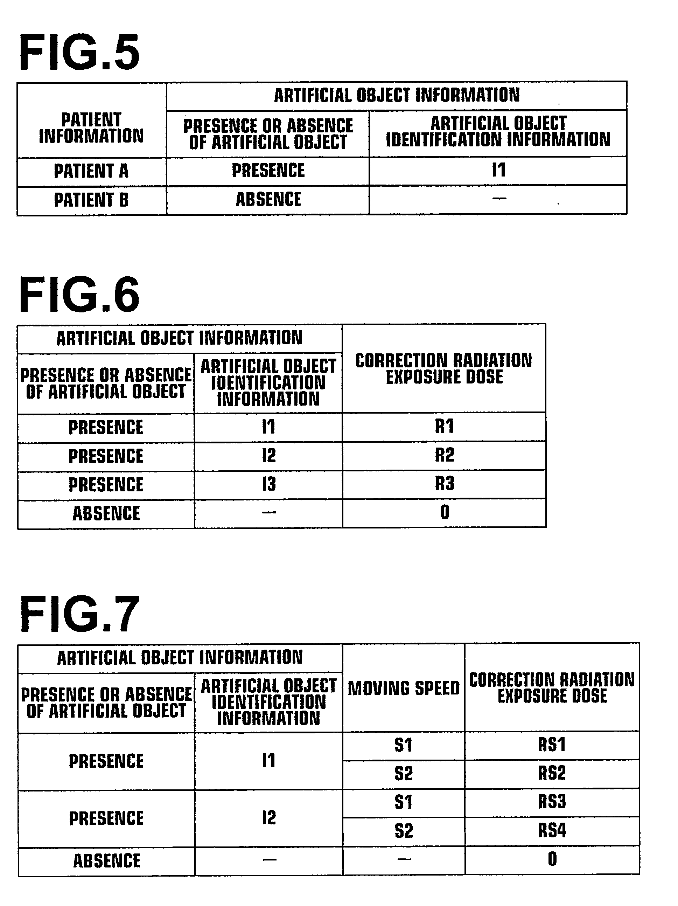

More specifically, based on the inputted patient information, artificial object information corresponding to the patient information is obtained first in the artificial object information obtaining unit 31 (S22). Further specifically, a table in which the patient information and artificial object information are associated, as illustrated in Figure 5, is preset in the artificial object information obtaining unit 31, and the artificial object information obtaining unit 31 obtains the artificial object information corresponding to the patient of the imaging target with reference to the table. It is assumed that, in the present embodiment, information of presence or absence of artificial object that indicates whether or not an artificial object is included in the body of the patient and identification information for identifying an artificial object are preset as the artificial object information, as shown in Figure 5.

Next, the artificial object information obtained by the artificial object information obtaining unit 31 is outputted to the radiation exposure dose obtaining unit 32.

Here, a table in which the artificial object information and the correction radiation dose are related is preset in the radiation exposure dose obtaining unit 32 and radiation exposure dose obtaining unit 32 obtains a correction radiation exposure dose based on the inputted artificial object information and the aforementioned table (S24).

Then, the radiation exposure dose obtaining unit 32 obtains a total radiation exposure dose by performing a correction in which the radiation exposure dose calculated based on the radiation image signal of each frame and the correction radiation exposure dose are added (S26).

Then, the total radiation exposure dose obtained in the radiation exposure dose obtaining unit 32 is inputted to the radiation exposure dose management apparatus 40 and the radiation exposure dose management apparatus 40 registers the total radiation exposure dose with the already inputted and preset patient information (S28). Then, the radiation exposure dose management apparatus 40 displays the registered total radiation exposure dose displays the registered total radiation exposure dose with the patient information as required or calculates a cumulative radiation exposure dose from the past for a particular patient and displays a warning message if the cumulative radiation exposure dose is greater than a predetermined specified value.

According to the radiation image capturing system of the present embodiment, in the case where a radiation exposure dose of a patient who includes an artificial object is obtained based on a radiation image signal detected by the radiation image detector 12 through the application of radiation transmitted through the patient, information of the artificial object included in the body of the patient is obtained, and in the case where information indicating that an artificial object is included in the body of the patient is obtained as the information of the artificial object, a correction to increase the radiation exposure dose, which is based on the radiation image signal, by a given correction radiation exposure dose is performed. This allows a more accurate radiation exposure dose of a human body which takes into account the radiation absorption by the artificial object to be obtained.

In the radiation image capturing system of the aforementioned embodiment, a constant correction radiation exposure dose is obtained for given artificial object information regardless of the movement speed of the radiation application unit 11 and the radiation image detector 12. But, for example, if the travel distance of the radiation application unit 11 and the radiation image detector 12 and the frame rate are constant, the number of frames varies with the movement speed of the radiation application unit 11 and the radiation image detector 12 and the magnitude of the correction radiation exposure dose to be corrected also varies. Therefore, a table in which the artificial object information, moving speed, and correction radiation exposure dose are related, as shown in Figure 6, may be preset and a correction exposure dose that also takes into account the movement speed information may be obtained based on the moving speed included in the imaging menu.

Further, if the travel distance and movement speed of the radiation application unit 11 and the radiation image detector 12 are constant, the number of frames varies with the frame rate and the magnitude of the correction radiation exposure dose to be corrected also varies. Therefore, a table in which the artificial object information, frame rate, and correction radiation exposure dose are related, as shown in Figure 7, may be preset and a correction exposure dose that also takes into account the movement speed may be obtained based on the frame rate information included in the imaging menu.

Further, a correction radiation exposure dose that takes into account both the movement speed of the radiation application unit 11 and the radiation image detector 12, and the frame rate may be preset, and still further, a correction radiation exposure dose that also takes into account the travel distance of the radiation application unit 11 and the radiation image detector 12 may be preset.

In the radiation image capturing system of the embodiment described above, a correction is made by adding a correction radiation exposure dose to the radiation exposure dose calculated based on the radiation image signal, but not limited to this and, for example, the correction may be made in which the radiation exposure dose calculated based on the radiation image signal is multiplied by a coefficient greater than 1 so as to be increased by a correction exposure dose.

Further, in the radiation image capturing system of the embodiment described above, the artificial object information obtaining unit 31 obtains artificial object information based on patient information included in the imaging menu, but not limited to this and an arrangement may be adopted in which input of artificial object information is received at the input unit 60 and the received artificial object information is obtained by the artificial object information obtaining unit 31.

As for the information of whether or not an artificial object is included in the body of a patient, an arrangement may be adopted in which, for example, a sensor capable of selectively detecting an artificial object included in the body of the patient is provided in the image capturing platform 16 in the radiation application unit 11 or the like and the information of presence or absence of artificial object is obtained based on the detection signal outputted from the sensor. Further, a mark representing artificial object information may be provided in advance and the artificial object identification information may be obtained through image recognition of the mark. Further, the mark may include, for example, information related to absorption of radiation, such as the material, thickness, and shape of the artificial object while a correction radiation exposure dose corresponding to the aforementioned information is set in the radiation exposure dose obtaining unit 32 in advance. Then, a correction may be made by the radiation exposure dose obtaining unit 32 by adding the correction radiation exposure dose corresponding to the obtained information described above to the radiation exposure dose calculated based on the radiation image signal of each frame.

Still further, the radiation image capturing system of the embodiment described above captures a moving picture by moving the radiation application unit 11 and the radiation image detector 12, but not limited to this and the present invention is also applicable to a radiation image capturing system that captures a moving picture with the radiation application unit 11 and the radiation image detector 12 being fixed. Still further, the present invention is not necessarily limited to those that capture moving pictures and also applicable to those that capture still images.

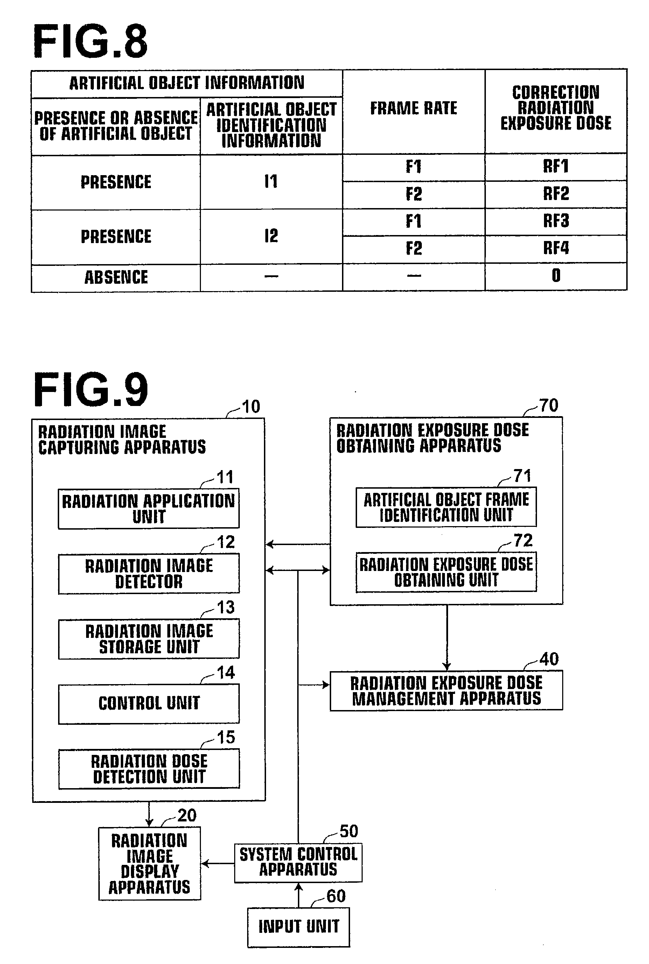

Next, a radiation image capturing system that uses a second embodiment of the radiation exposure dose obtaining apparatus of the present invention will be described. Figure 9 is a block diagram of the radiation image capturing system of the present embodiment, illustrating an overall schematic configuration thereof.

As illustrated in Figure 1, the radiation image capturing system of the present embodiment includes a radiation image capturing apparatus 10, a radiation image display apparatus 20, a radiation exposure dose obtaining apparatus 70, a radiation exposure dose management apparatus 40, and a system control apparatus 50. The radiation image display apparatus 20, the radiation exposure dose management apparatus 40, the system control apparatus 50, and the input unit 60 are identical to those of the radiation image capturing system of the first embodiment described above.

In the radiation image capturing system of the present invention, the configurations of the radiation image capturing apparatus 10 and the radiation exposure dose obtaining apparatus 70 are different from those of the radiation image capturing system of the first embodiment described above. Therefore, the description will be made by focusing the differences from the radiation image capturing system of the first embodiment.

The radiation image capturing apparatus 10 of the present embodiment includes the radiation application unit 11, the radiation image detector 12, the radiation image storage unit 13, and control unit 14, as in the first embodiment. In addition, the apparatus 10 includes a radiation dose detection unit 15 provided between a patient and the radiation image detector 12 to detect the dose of radiation transmitted through the patient.

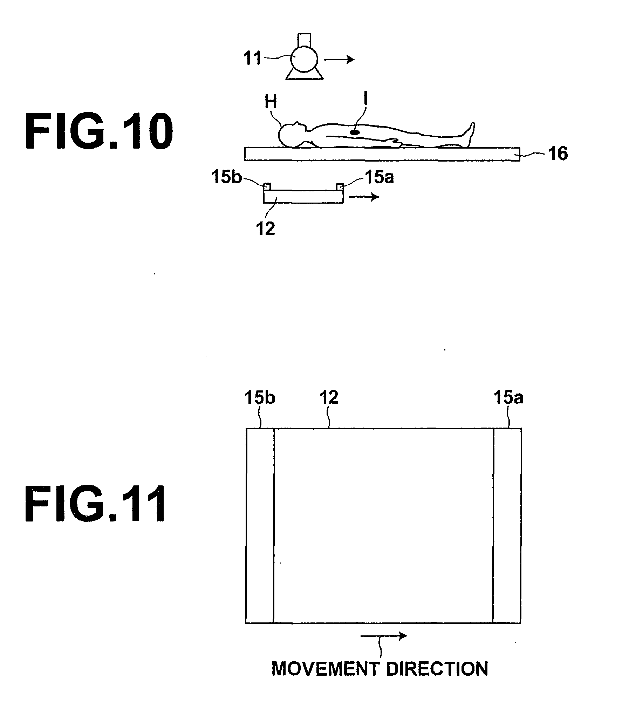

As described above, the radiation dose detection unit 15 is provided between a patient and the radiation image detector 12, and in the present embodiment, the radiation dose detection unit 15 includes a first dose measurement sensor 15a and a second dose measurement sensor 15b provided on the radiation receiving surface of the radiation image detector 12, as illustrated in Figures 10 and 11. Figure 11 is a view of the radiation image detector 12 and the first and second dose measurement sensors 15a, 15b shown in Figure 10 viewed from above.

As illustrated in Figure 11, the first dose measurement sensor 15a and the second measurement sensor 15b are provided along the opposite sides in the movement direction of the radiation image detector 12. In the present embodiment, dose measurement sensors are provided along only the opposite sides in the movement direction as described above, but it is more preferable that dose measurement sensors are provided along the four sides of the radiation image detector 12.

As for the first and second dose measurement sensors 15a, 15b, the use of a thin sensor with substantially no radiation absorption is preferable and, for example, a sensor made of an organic photoelectric conversion material (OPC) is preferably used.

If a radiation image detector formed of a scintillator layer that converts radiation to visible light and a sensor substrate provided with TFT or CMOS switches that detect the light emitted from the scintillator layer layered on top of each other is used as the radiation image detector 12, it is preferable that the scintillator layer is disposed opposite to the radiation receiving side. That is, it is preferable that the first and second dose measurement sensors 15a, 15b, the sensor substrate, and the scintillator layer are arranged in this order from the radiation receiving side.

As the light emitting portion of the scintillator layer is closer to the radiation receiving side, the distance between the light emitting portion of the scintillator layer and the sensor substrate may be reduced by arranging them in the manner described above. This may inhibit blurring of radiation images due to diffusion of light emitted from the scintillator layer and higher signal intensity may be obtained.

The configuration of the radiation image detector 12 described above is not limited to the second embodiment and may be adopted in the other embodiment described above.

In the present embodiment, the first and second dose measurement sensors 15a, 15b are provided on the radiation image detector 12, but not limited to this and they may be set at the other predetermined position as long as it is between the patient and radiation image detector 12.

The radiation dose detection unit 15 sequentially outputs information of the detected dose of radiation to an artificial object frame identification unit 71, to be described later, in parallel with fluoroscopic image capturing.

The structure of the radiation image capturing apparatus 10 is not limited to the structure in which image capturing is performed with the patient being in the lateral position, and the structure in which image capturing is performed with the patient being in the upright position, as in the first embodiment. Further, a structure that allows image capturing with the patient being both in upright position and lateral position may be employed.

The radiation exposure dose obtaining apparatus 70 includes an artificial object frame identification unit 71 that identifies a radiation image signal of a frame which includes an image signal of an image of an artificial object as an artificial object frame from the radiation image signal of each frame read out from the radiation image storage unit 13 of the radiation image capturing apparatus 10, and a radiation exposure dose obtaining unit 72 that obtains a radiation exposure dose of the patient based on the radiation image signal of each frame read out from the radiation image storage unit 13 of the radiation image capturing apparatus 10.

The artificial object frame identification unit 71 identifies an artificial object frame from a plurality of frames obtained by the fluoroscopic image capturing based on information of radiation dose outputted from the first dose measurement sensor 15a and the second dose measurement sensor 15b constituting the radiation dose detection unit 15.

Further, the artificial object frame identification unit 71 of the present embodiment stores the radiation image signal of a frame determined to be an artificial object frame after appending information indicating that it is an artificial object frame.

Operations of the artificial object frame identification unit 71 and the radiation exposure dose obtaining unit 72 will be described later in detail.

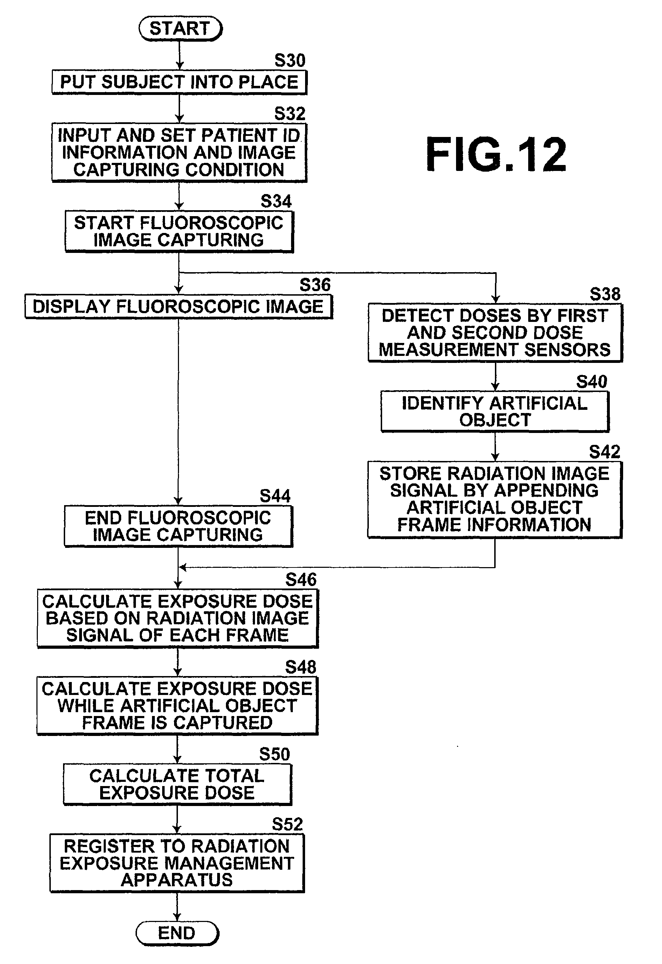

An operation of the radiation image capturing system of the present embodiment will now be described with reference to the flowchart of Figure 12.

First, a patient is placed on an image capturing platform 16 provided in the radiation image capturing apparatus 10 and the patient is put into position (S30).

Then, ID information of the image capturing target patient and a given image capturing condition are inputted by the user using the input unit 60 and the patient ID information is registered in the radiation exposure dose management apparatus 40 while the image capturing condition is set to the control unit 14 of the radiation image capturing apparatus 10 (S32). The image capturing condition may include a tube voltage, tube current, and application time in order to apply an appropriate dose of radiation to an image capturing region of the patient, as well as a frame rate for capturing a fluoroscopic image. As for the frame rate for capturing the fluoroscopic image, for example, a frame rate of 5fps to 60fps is set.

Next, an instruction to start fluoroscopic image capturing of the patient is inputted by the user using the input unit 60 and in response to the input, a control signal is outputted from the system control apparatus 50 to the radiation image capturing apparatus 10 to capture a fluoroscopic image, and the radiation image capturing apparatus 10 starts fluoroscopic image capturing according to the inputted control signal (S34).

More specifically, the radiation application unit 111 and the radiation image detector 12 are moved relative to the patient in response to the inputted control signal and the X-ray tube of the radiation application unit 11 is controlled based on the inputted imaging condition and a predetermined dose of radiation is applied toward the patient intermittently at a predetermined frame rate.

Then, radiation transmitted through the patient is applied to the radiation image detector 12 and subjected to a photoelectrical conversion in the radiation image detector 12 and accumulated therein as a charge signal.

Thereafter, each time the application of radiation for each frame is ended, the charge signal accumulated in the radiation image detector 12 is read out by the control unit 14, then converted to a digital signal by an A/D converter (not shown), and stored in the radiation image storage unit 13. Note that the application timing of radiation from the X-ray tube of the radiation application unit 11 and the charge accumulation timing of the radiation image detector 12 are as in the timing chart of the first embodiment shown in Figure 4.

The repeated performance of radiation application by the X-ray tube and the radiation image recording and reading in the radiation image detector 12 at a predetermined frame rate in the manner described above causes the radiation image signal of each frame to be sequentially stored in the radiation image storage unit 13.

Then, the radiation image signal of each frame stored in the radiation image storage apparatus 13 is sequentially read out and outputted to the radiation image display apparatus 20. The radiation image display apparatus 20 sequentially generates a display control signal based on the inputted radiation image signal of each frame and sequentially outputs the display control signal to the monitor to display a fluoroscopic image of the patient as a moving picture (S36).

In the mean time, the dose of radiation transmitted through the patient is sequentially detected by the first and second dose measurement sensors 15a, 15b provided on the radiation image detector 12 in parallel with the fluoroscopic image capturing and display described above, and the detected dose of radiation is sequentially inputted to the artificial object frame identification unit 71 of the radiation exposure dose obtaining apparatus 30 (S38).

Then, the artificial object frame identification unit 71 obtains a temporal variation in the inputted dose of radiation, and identifies an artificial object frame in which an image of an artificial object is imaged based on the temporal variation (S40) .

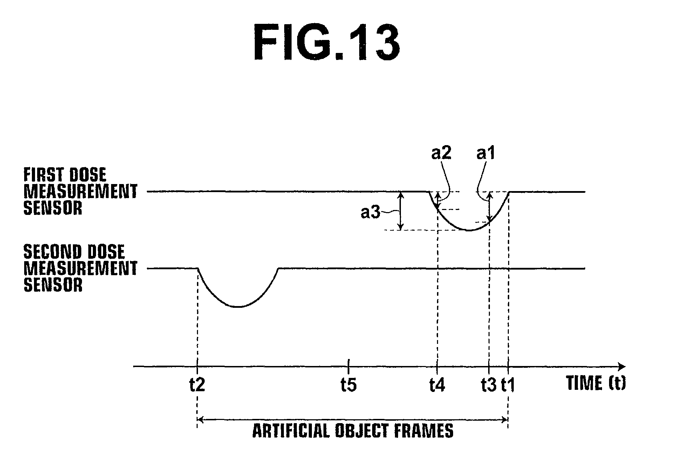

More specifically, the artificial object frame identification unit 71 obtains, based on the dose of radiation detected by the first dose measurement sensor 15a and the dose of radiation detected by the second dose measurement sensor 15b, a temporal variation in the dose of radiation detected by each sensor, as illustrated in Figure 13.

Here, when an artificial object embedded in the patient passes over the first and second dose measurement sensors 15a, 15b, the radiation is absorbed by the artificial object and the dose of radiation detected by each sensor is reduced, as illustrated in Figure 13.

By using such a change, the artificial object frame identification unit 71 identifies a frame captured from a first time point t1 at which the dose of radiation detected by the first dose measurement sensor 15a disposed in the downstream begins to decrease to a time point t2 at which the dose of radiation detected by the second dose measurement sensor 15b disposed in the upstream once decreased and then returned to a substantially constant value as an artificial object frame. It is assumed here that the movement speed of the radiation application unit 11 and the radiation image detector 12 and the frame rate of radiological imaging of the fluoroscopic image are preset in the artificial object frame identification unit 71, and the artificial object frame identification unit 71 identifies an artificial object frame based on the information of these and the time from the first time point t1 to the second time point t2.

The radiation image signal read out from the radiation image storage unit 13 is inputted also to the artificial object frame identification unit 71, and the artificial object frame identification unit 71 appends, to the radiation image signal of the frame identified as an artificial object frame in the manner described above, information indicating that it is an artificial object frame (hereinafter, referred to as "artificial object frame information") as header information and stores the radiation image signal with the artificial object frame information (S42).

Thereafter, when an instruction to end the fluoroscopic image capturing is inputted by the user using the input unit 60, the system control apparatus 50 outputs a control signal to the radiation image capturing apparatus 10 to end the fluoroscopic image capturing, and the radiation image capturing apparatus 10 ends the fluoroscopic image capturing in response to the inputted control signal (S44).

When the fluoroscopic image capturing described above is ended, a radiation exposure dose of the patient is obtained in the radiation exposure dose obtaining apparatus 70.

More specifically, the radiation image signal of each frame stored in the artificial object frame identification unit 71 is outputted to the radiation exposure dose obtaining unit 72 with the artificial object frame information.

Next, a radiation exposure dose received by the patient during imaging of each frame of the fluoroscopic image is calculated in the radiation exposure dose obtaining unit 72 based on the radiation image signal of each frame (S46). More specifically, an E.I. is calculated based on the radiation image signal of each frame and a radiation exposure dose is obtained based on the E.I. also in the present embodiment, as in the first embodiment. The calculation method of E.I. and radiation exposure dose obtaining method base on the E.I. are as explained in the first embodiment.

Here, if a fluoroscopic image of a patient with an embedded artificial object is captured, the radiation applied to the patient is absorbed by the artificial object before reaching the radiation image detector 12. Therefore, if a radiation exposure dose is calculated based on the radiation image signal of the artificial object frame, the calculated value will become smaller than that actually received by the patient.

Consequently, in the present embodiment, actual radiation exposure doses of the patient during the capturing of artificial object frames are obtained by obtaining radiation exposure doses corrected based on the information of artificial object frames identified in the artificial object frame identification unit 71 (S48).

More specifically, the radiation exposure dose obtaining unit 72 corrects the radiation exposure dose calculated based on the radiation image signal of the artificial object frame by adding a predetermined correction exposure dose. In the present embodiment, dose information detected by the first or second dose measurement sensor 105a or 105b during the correction target artificial object frame is captured as the correction exposure dose. That is, the decrease in the detected dose by each sensor due to radiation absorption by the artificial object is used as the correction exposure dose. For example, the radiation exposure dose corresponding to the artificial object frame captured at the time point t3 in Figure 13 is corrected by adding the correction exposure dose a1. The radiation exposure dose corresponding to the artificial object frame captured at the time point t4 in Figure 13 is corrected by adding the correction exposure dose a2. For radiation doses corresponding to artificial object frames captured while the artificial object is passing between the first dose measurement sensor 15a and the second dose measurement sensor 15b, a maximum correction dose a3 when the detection dose of each sensor is decreased the most is added. That is, for example, the radiation exposure dose corresponding to the artificial object frame captured at the time point t5 in Figure 13 is corrected by adding the correction exposure dose a3.

Next, the radiation exposure dose obtaining unit 72 calculates the total radiation exposure dose received by the patient by adding the radiation exposure doses during the capturing of the artificial obj ect frames obtained in the manner described above to the radiation exposure doses during the capturing of frames other than the artificial object frames (S50).

The total radiation exposure dose obtained in the radiation exposure dose obtaining unit 72 in the manner described above is inputted to the radiation exposure dose management apparatus 40, and the radiation exposure dose management apparatus 40 registers the total radiation exposure dose with the ID information of the patient inputted in advance (S52) . Then, the radiation exposure dose management apparatus 40 displays the registered total radiation exposure dose with the ID information of the patient as required or calculates a cumulative radiation exposure dose from the past for a particular patient and displays a warning message if the cumulative radiation exposure dose is greater than a predetermined specified value.

According to the radiation image capturing system of the second embodiment, the radiation exposure dose of a living body is obtained for the radiological imaging of an artificial object by identifying a frame which includes an image signal representing an image of an artificial object as an artificial object frame from a plurality of frames based on dose information of radiation detected by the radiation dose detection unit 15 provided between the patient and radiation image detector 12, and correcting the radiation exposure dose based on the information of the identified artificial object frame. This allows appropriate identification of an artificial object frame to be made by a simple structure. Further, a radiation exposure dose throughout the radiological imaging which takes into account the inclusion of an image of an artificial object in the radiation image may be obtained more accurately.

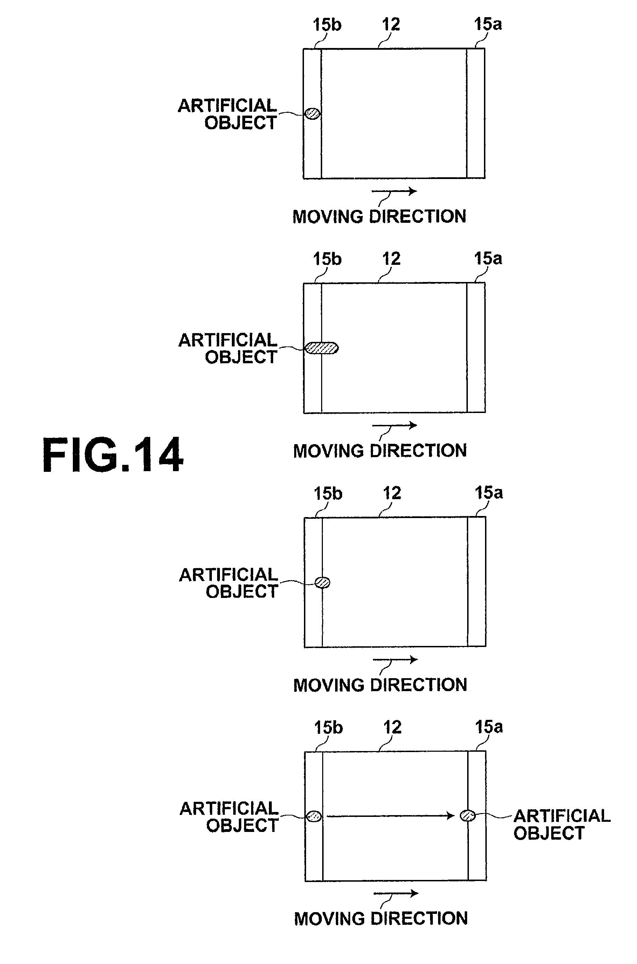

In the radiation image capturing system of the embodiment described above, when calculating a radiation exposure dose corresponding to an artificial object frame, a decrease in the detected dose of the first or second dose measurement sensor 15a or 15b is used as the correction radiation exposure dose. In such a method, if the artificial object is imaged at the timing when the artificial object falls within the first or the second dose measurement sensor 15a or 15b as illustrated, for example, in the top of Figure 14, a correction radiation exposure dose appropriate for the actual radiation exposure dose of the patient may be calculated. If the artificial object is larger than the first or second dose measurement sensor 15a or 15b, however, an artificial object frame may be captured with the artificial object being protruded from the first or second dose measurement sensor 15a or 15b, as illustrated in the second drawing from the top of Figure 14. In such a case, the correction radiation exposure dose is reduced by the amount corresponding to the portion protruded from the first or second dose measurement sensor 15a or 15b.

Further, even in the case where the artificial object is smaller than the first or second dose measurement sensor 15a or 15b, an artificial object frame may possibly be captured at the timing at which the artificial object is protruded from the first or second dose measurement sensor 15a or 15b, as illustrated in the third and fourth drawings from the top of Figure 14. In this case also, the correction radiation exposure dose is reduced by the amount corresponding to the portion protruded from the first or second dose measurement sensor 15a or 15b, as in the case described above.

Consequently, an arrangement may be adopted in which, for the first artificial object frame or several artificial object frames from the first, and for the last artificial object frame or several artificial object frames from the last, a radiation image captured by the radiation image detector 12 corresponding to each of the frames is referenced and a correction radiation exposure dose is calculated based on the size of the artificial object imaged in the radiation image or the amount protruded from the first or second dose measurement sensor 15a or 15b.

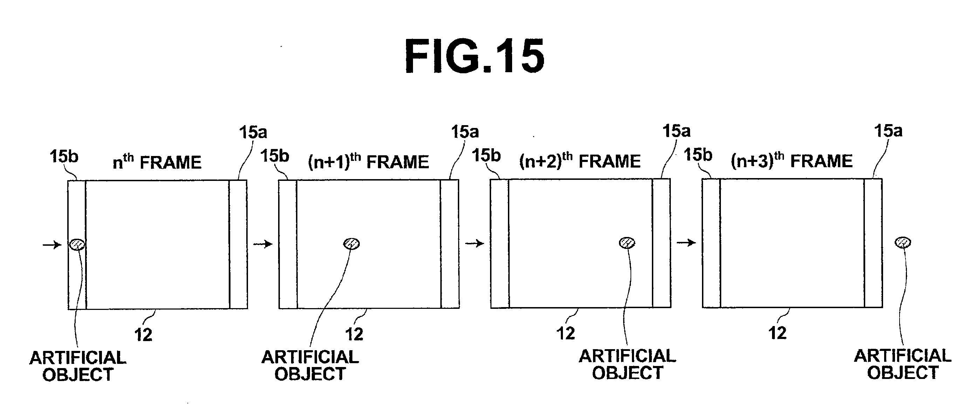

Further, depending on the movement speed of the radiation image detector 12 and the frame rate of the radiation image capturing, there may be a case in which an artificial object is detected by the second dose measurement sensor 15b while it is not detected by the first dose measurement sensor 15a or vice versa as illustrated in Figure 15.

In such a case, the number of artificial object frames is not known because the end of the artificial object frames can not be detected though the start thereof is detected or vice versa.

Consequently, in such a case, for example, in the case illustrated in Figure 15, from $n^{th}$ frame to $(n+2)^{th}$ frame may be identified as artificial object frames based on the movement speed of the radiation image detector 12 and the frame rate of the radiation image capturing. Contrary to this, if only the last artificial object frame is detected, artificial object frames may be identified by counting the number of artificial object frames captured before the last frame based on the movement speed of the radiation image detector 12 and the frame rate of the radiation image capturing. In this way, the artificial object frames may be identified and an accurate radiation exposure dose may be calculated. If the start or end of artificial object frames can not be detected, as described above, the artificial object frames may be identified by referencing to the radiation images detected by the radiation image detector 12.

Further, in the radiation image capturing system of the embodiment described above, the radiation exposure dose is calculated after the fluoroscopic image capturing is completed, but not limited to this and the radiation exposure dose is calculated in the manner described above in the middle of the fluoroscopic image capturing and the radiation exposure dose of a subject in the middle of the imaging may be displayed or the like.

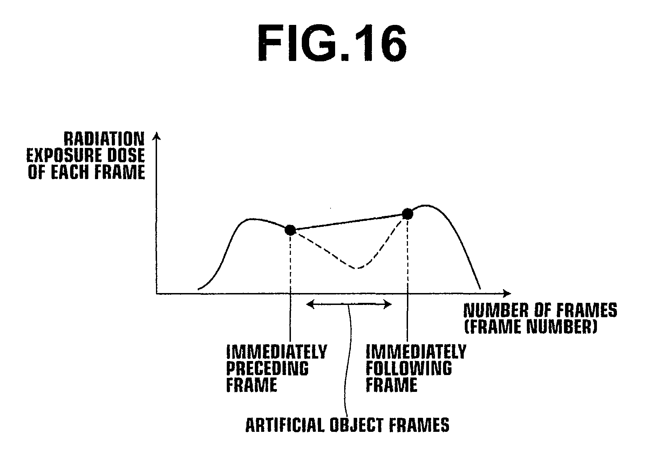

Further, in the radiation image capturing system of the embodiment described above, the correction is performed by adding a correction exposure dose to the radiation exposure dose calculated based on the radiation image signal of the artificial object frame, but the method for obtaining a radiation exposure dose during imaging of an artificial object is not limited to this and, for example, a radiation exposure dose of the patient during imaging of an artificial object may be obtained through linear interpolation of radiation exposure doses calculated based on the frames immediately preceding and immediately following the artificial object frame, as illustrated in Figure 16.

Further, a radiation exposure dose calculated based on the radiation image signal of the frame immediately preceding or immediately following the artificial object frame may be employed directly as the radiation exposure dose during the artificial object frame capturing, or a radiation exposure dose calculated based on the radiation image signal of another frame other than the artificial object frame may be employed. Otherwise, a radiation exposure dose calculated based on the radiation image signal of an artificial object frame may be corrected by adding a predetermined radiation exposure dose determined in advance or by multiplying it with a given coefficient which is greater than 1.

Still further, in the radiation image capturing system of the embodiment described above, an artificial object frame is identified based on the temporal variations in the doses of radiation detected by the first and second dose measurement sensors 15a, 15b, but not limited to this and, for example, an index, such as a mark, indicating that it is an artificial object may be provided on an artificial object to be embedded in the body of a patient, and an artificial object frame may be identified by the artificial object frame identification unit 71 through image recognition as to whether or not an image signal representing the aforementioned index is included in the radiation image signal of each frame. As the mark image recognition is an already known technique, it will not be elaborated upon further here.

In the case where an index is provided for an artificial object as described above, the index may include not only the information that indicates that it is an artificial object but also information related to correction of the radiation exposure dose. Then, a radiation exposure dose corresponding to the artificial object frame may be corrected by the radiation exposure dose obtaining unit 72 by obtaining the information related to the correction included in the index. More specifically, the index may include, for example, information related to absorption of radiation, such as the material, thickness, and shape of the artificial object while a correction radiation exposure dose corresponding to the aforementioned information is set in the radiation exposure dose obtaining unit 72 in advance. Then, a correction may be made by the radiation exposure dose obtaining unit 72 by adding the correction radiation exposure dose corresponding to the obtained information described above to the radiation exposure dose calculated based on the radiation image signal of the artificial object frame.

Further, in the first and second embodiments, the radiation exposure dose obtaining apparatus is implemented as an independent apparatus, but it may be implemented in any other form, such as being incorporated in the other apparatus, such as the radiation image capturing apparatus, as a part thereof. More specifically, it may be provided in a console which includes the system control apparatus 50 or in the radiation image capturing apparatus 10. Further, in the case where the radiation image detector 12 is accommodated in a portable electronic cassette, and hardware of electronic circuits, such as LSI (Large Scale Integration), hardware of programmable electronic circuits, such as PLD (Programmable Logic Device) · FPGA (Field-Programmable Gate Array), and the like are accommodated in the electronic cassette, the identification of an artificial object frame and acquisition of the radiation exposure dose may be performed by such hardware. Such arrangement allows pursuit of more real time implementation.

## Claims

1. A radiation exposure dose obtaining apparatus, comprising:

   an artificial object information obtaining unit that obtains information of an artificial object included in a human body which is an imaging target of radiological imaging; and
   a radiation exposure dose obtaining unit that obtains a radiation exposure dose of the human body based on a radiation image signal detected by a radiation image detector through the application of radiation transmitted through the human body,

wherein, in the case where information indicating that an artificial object is included in the human body is obtained by the artificial obj ect information obtaining unit, the radiation exposure dose obtaining unit performs a correction to increase the radiation exposure dose, which is based on the radiation image signal, by a predetermined correction radiation exposure dose.

2. The radiation exposure dose obtaining apparatus of claim 1, wherein:

a correspondence relationship between identification information of the human body and information of the artificial object of the human body is preset; and
the artificial object information obtaining unit receives identification information of the human body and obtains information of the artificial object of the human body corresponding to the received identification information of the human body.

3. The radiation exposure dose obtaining apparatus of claim 2, wherein the identification information of the human body is included in an imaging menu of the radiological imaging.

4. The radiation exposure dose obtaining apparatus of any of claims 1 to 3, wherein:

the artificial object information obtaining unit obtains artificial object identification information for identifying the artificial object as the information of the artificial object; and
the radiation exposure dose obtaining unit includes a table in which the artificial object identification information and the correction radiation dose are related and performs the correction based on the artificial object identification information obtained by the artificial object information obtaining unit and the table.

5. The radiation exposure dose obtaining apparatus of any of claims 1 to 4, wherein the radiation exposure dose obtaining unit obtains the radiation exposure dose of the human body based on a radiation image signal of each frame detected by the radiation image detector through a continuous application of the radiation to the human body and performs a correction to increase by a correction radiation exposure dose according to the frame rate of the radiation image signal.

6. The radiation exposure dose obtaining apparatus of any of claims 1 to 5, wherein the radiation exposure dose obtaining unit obtains the radiation exposure dose of the human body based on a radiation image signal of each frame detected by the radiation image detector through a continuous application of the radiation to the human body while moving the application range of the radiation with respect to the human body, and performs a correction to increase by a correction radiation exposure dose according to the movement speed of the application range of the radiation.

7. A radiation image capturing system, comprising:

the radiation exposure dose obtaining apparatus of claim 3; and
a radiation image capturing apparatus that obtains the radiation image signal by performing the radiological imaging,
wherein the radiation image capturing apparatus performs the radiological imaging based on the imaging menu that includes the identification information of the human body.

8. A radiation exposure dose obtaining method that obtains, based on a radiation image signal detected by a radiation image detector through the application of radiation transmitted through a human body that includes an artificial object, a radiation exposure dose of the human body, the method comprising the steps of:

obtaining information of the artificial object included in the human body; and
performing, in the case where information indicating that an artificial object is included in the human body is obtained as the information of the artificial object, a correction to increase the radiation exposure dose, which is based on the radiation image signal, by a predetermined correction radiation exposure dose.

9. A radiation exposure dose obtaining apparatus, comprising:

a radiation exposure dose obtaining unit that obtains, based on a radiation image signal of each frame detected by a radiation image detector through continuous radiological imaging of a living body that includes an artificial object while moving the application range of the radiation relative to the living body, a radiation exposure dose of the living body due to the continuous radiological imaging; and
an artificial object frame identification unit that identifies a frame that includes an image signal representing an image of the artificial object as an artificial object frame from a plurality of frames,

wherein the radiation exposure dose obtaining unit corrects the radiation exposure dose based on information of the artificial object frame and obtains a radiation exposure dose of the living body for the radiological imaging of the artificial object frame.

10. The radiation exposure dose obtaining apparatus of claim 9, wherein the artificial object frame identification unit identifies the artificial object frame based on dose information of radiation detected by a radiation dose detection unit provided between the living body and the radiation image detector.

11. The radiation exposure dose obtaining apparatus of claim 10, wherein the artificial object frame identification unit obtains a temporal variation in the dose information of radiation while the continuous radiological imaging is performed.

12. The radiation exposure dose obtaining apparatus of claim 10 or 11, wherein the radiation exposure dose obtaining unit corrects the radiation exposure dose obtained based on the radiation image signal of the artificial object frame using the dose information of radiation detected by the radiation dose detection unit and obtains a radiation exposure dose of the living body for the radiological imaging of the artificial object frame.

13. The radiation exposure dose obtaining apparatus of claim 12, wherein the radiation exposure dose obtaining unit corrects the radiation exposure dose obtained based on the radiation image signal of the artificial object frame using the temporal variation in the dose information of radiation, the frame rate of the radiological imaging, and the speed of the movement.

14. The radiation exposure dose obtaining apparatus of any of claims 10 to 13, wherein the radiation dose detection unit is provided on the radiation image detector.

15. The radiation exposure dose obtaining apparatus of claim 14, wherein the radiation dose detection unit is provided at a peripheral portion of the radiation image detector.

16. The radiation exposure dose obtaining apparatus of claim 15, wherein the radiation dose detection unit is provided on each of at least two opposite sides of the radiation image detector in the direction of the movement.

17. The radiation exposure dose obtaining apparatus of claim 9, wherein:

the artificial object is provided with an index indicating as being an artificial object; and
the artificial object identification unit identifies the artificial object by recognizing an image signal of the index included in the radiation image signal of each frame.

18. The radiation exposure dose obtaining apparatus of claim 17, wherein:

the index includes information used for the correction of the radiation exposure dose; and
the radiation exposure dose obtaining unit performs the correction of the radiation exposure dose based on the information included in the index.

19. A radiation exposure dose obtaining method that obtains, based on a radiation image signal of each frame detected by a radiation image detector through continuous radiological imaging of a living body that includes an artificial object while moving the application range of the radiation relative to the living body, a radiation exposure dose of the living body due to the continuous radiological imaging, the method comprising the steps of:

identifying a frame that includes an image signal representing an image of the artificial object as an artificial object frame from a plurality of frames; and
correcting the radiation exposure dose based on information of the identified artificial object frame and obtaining a radiation exposure dose of the living body for the radiological imaging of the artificial object frame.

# FIG.1

**RADIATION IMAGE CAPTURING APPARATUS** `10`

> **RADIATION APPLICATION UNIT** `11`
>
> **RADIATION IMAGE DETECTOR** `12`
>
> **RADIATION IMAGE STORAGE UNIT** `13`
>
> **CONTROL UNIT** `14`

**RADIATION EXPOSURE DOSE OBTAINING APPARATUS** `30`

> **ARTIFICIAL OBJECT INFORMATION OBTAINING UNIT** `31`
>
> **RADIATION EXPOSURE DOSE OBTAINING UNIT** `32`

**RADIATION EXPOSURE DOSE MANAGEMENT APPARATUS** `40`

**RADIATION IMAGE DISPLAY APPARATUS** `20`

**SYSTEM CONTROL APPARATUS** `50`

**INPUT UNIT** `60`

# FIG.2

# FIG.3

START

↓

PUT SUBJECT INTO PLACE — S10

↓

INPUT AND SET IMAGING MENU
INCLUDING PATIENT INFORMATION — S12

↓

START FLUOROSCOPIC IMAGE CAPTURING — S14

↓

DISPLAY FLUOROSCOPIC IMAGE — S16

↓

END FLUOROSCOPIC IMAGE CAPTURING — S18

↓

CALCULATE RADIATION EXPOSURE DOSE BASED
ON RADIATION IMAGE SIGNAL OF EACH FRAME — S20

↓

OBTAIN ARTIFICIAL OBJECT INFORMATION
RELATED TO PATIENT INFORMATION — S22

↓

OBTAIN CORRECTION EXPOSURE DOSE CORRESPONDING
TO ARTIFICIAL OBJECT INFORMATION — S24

↓

CORRECT EXPOSURE DOSE — S26

↓

REGISTER TO RADIATION EXPOSURE DOSE
MANAGEMENT APPARATUS — S28

↓

END

# FIG.4

X-RAY OUTPUT
OF X-RAY TUBE

CHARGE ACCUMULATION
OF RADIATION IMAGE DETECTOR

FRAME

ON
OFF

ACCUMULATION
ON
ACCUMULATION
OFF

# FIG.5

| PATIENT INFORMATION | ARTIFICIAL OBJECT INFORMATION | |
|---|---|---|
| | PRESENCE OR ABSENCE OF ARTIFICIAL OBJECT | ARTIFICIAL OBJECT IDENTIFICATION INFORMATION |
| PATIENT A | PRESENCE | I1 |
| PATIENT B | ABSENCE | — |

# FIG.6

| ARTIFICIAL OBJECT INFORMATION | | CORRECTION RADIATION EXPOSURE DOSE |
|---|---|---|
| PRESENCE OR ABSENCE OF ARTIFICIAL OBJECT | ARTIFICIAL OBJECT IDENTIFICATION INFORMATION | |
| PRESENCE | I1 | R1 |
| PRESENCE | I2 | R2 |
| PRESENCE | I3 | R3 |
| ABSENCE | — | 0 |

# FIG.7

| ARTIFICIAL OBJECT INFORMATION | | MOVING SPEED | CORRECTION RADIATION EXPOSURE DOSE |
|---|---|---|---|
| PRESENCE OR ABSENCE OF ARTIFICIAL OBJECT | ARTIFICIAL OBJECT IDENTIFICATION INFORMATION | | |
| PRESENCE | I1 | S1 | RS1 |
| | | S2 | RS2 |
| PRESENCE | I2 | S1 | RS3 |
| | | S2 | RS4 |
| ABSENCE | — | — | 0 |

# FIG.8

| ARTIFICIAL OBJECT INFORMATION | | FRAME RATE | CORRECTION RADIATION EXPOSURE DOSE |
|---|---|---|---|
| PRESENCE OR ABSENCE OF ARTIFICIAL OBJECT | ARTIFICIAL OBJECT IDENTIFICATION INFORMATION | | |
| PRESENCE | I1 | F1 | RF1 |
| | | F2 | RF2 |
| PRESENCE | I2 | F1 | RF3 |
| | | F2 | RF4 |
| ABSENCE | — | — | 0 |

# FIG.9

RADIATION IMAGE CAPTURING APPARATUS ~10

RADIATION APPLICATION UNIT ~11

RADIATION IMAGE DETECTOR ~12

RADIATION IMAGE STORAGE UNIT ~13

CONTROL UNIT ~14

RADIATION DOSE DETECTION UNIT ~15

RADIATION EXPOSURE DOSE OBTAINING APPARATUS ~70

ARTIFICIAL OBJECT FRAME IDENTIFICATION UNIT ~71

RADIATION EXPOSURE DOSE OBTAINING UNIT ~72

RADIATION EXPOSURE DOSE MANAGEMENT APPARATUS ~40

RADIATION IMAGE DISPLAY APPARATUS ~20

SYSTEM CONTROL APPARATUS ~50

INPUT UNIT ~60

**FIG.10**

11

H          I

15b          15a

12          16

**FIG.11**

15b          12          15a

MOVEMENT DIRECTION

EP 2 716 221 A1

START

PUT SUBJECT INTO PLACE — S30

INPUT AND SET PATIENT ID INFORMATION AND IMAGE CAPTURING CONDITION — S32

START FLUOROSCOPIC IMAGE CAPTURING — S34

**FIG.12**

DISPLAY FLUOROSCOPIC IMAGE — S36

DETECT DOSES BY FIRST AND SECOND DOSE MEASUREMENT SENSORS — S38

IDENTIFY ARTIFICIAL OBJECT — S40

STORE RADIATION IMAGE SIGNAL BY APPENDING ARTIFICIAL OBJECT FRAME INFORMATION — S42

END FLUOROSCOPIC IMAGE CAPTURING — S44

CALCULATE EXPOSURE DOSE BASED ON RADIATION IMAGE SIGNAL OF EACH FRAME — S46

CALCULATE EXPOSURE DOSE WHILE ARTIFICIAL OBJECT FRAME IS CAPTURED — S48

CALCULATE TOTAL EXPOSURE DOSE — S50

REGISTER TO RADIATION EXPOSURE MANAGEMENT APPARATUS — S52

END

21

# FIG.13

15b  12  15a

ARTIFICIAL
OBJECT

MOVING DIRECTION

15b  12  15a

ARTIFICIAL
OBJECT

# FIG.14

MOVING DIRECTION

15b  12  15a

ARTIFICIAL
OBJECT

MOVING DIRECTION

15b  12  15a

ARTIFICIAL
OBJECT                    ARTIFICIAL
                          OBJECT

MOVING DIRECTION

# FIG.15

15b    n<sup>th</sup> FRAME    15a    15b    (n+1)<sup>th</sup> FRAME    15a    15b    (n+2)<sup>th</sup> FRAME    15a    15b    (n+3)<sup>th</sup> FRAME    15a

12    12    12    12

ARTIFICIAL
OBJECT

ARTIFICIAL
OBJECT

ARTIFICIAL
OBJECT

ARTIFICIAL
OBJECT

# FIG.16

RADIATION
EXPOSURE DOSE
OF EACH FRAME

NUMBER OF FRAMES
(FRAME NUMBER)

IMMEDIATELY
PRECEDING
FRAME

IMMEDIATELY
FOLLOWING
FRAME

ARTIFICIAL OBJECT FRAMES

<div align="center">**INTERNATIONAL SEARCH REPORT**</div>

| International application No. |
| --- |
| PCT/JP2012/003464 |

A. CLASSIFICATION OF SUBJECT MATTER
*A61B6/00*(2006.01)i

According to International Patent Classification (IPC) or to both national classification and IPC

B. FIELDS SEARCHED

Minimum documentation searched (classification system followed by classification symbols)
A61B6/00

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2004-65815 A (Toshiba Medical Systems Engineering Co., Ltd.), 04 March 2004 (04.03.2004), entire text; all drawings (Family: none) | 1-19 |
| A | JP 2005-270286 A (Toshiba Corp.), 06 October 2005 (06.10.2005), entire text; all drawings (Family: none) | 1-19 |
| A | JP 2005-296277 A (Toshiba Corp.), 27 October 2005 (27.10.2005), entire text; all drawings (Family: none) | 1-19 |

☒ Further documents are listed in the continuation of Box C. ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>26 September, 2012 (26.09.12) | Date of mailing of the international search report<br>09 October, 2012 (09.10.12) |
| --- | --- |
| Name and mailing address of the ISA/<br>Japanese Patent Office | Authorized officer |
| Facsimile No. | Telephone No. |

Form PCT/ISA/210 (second sheet) (July 2009)

<div align="center">26</div>

**INTERNATIONAL SEARCH REPORT**

International application No.

PCT/JP2012/003464

C (Continuation).  DOCUMENTS CONSIDERED TO BE RELEVANT

| Category* | Citation of document, with indication, where appropriate, of the relevant passages | Relevant to claim No. |
|---|---|---|
| A | JP 2007-97909 A  (Kabushiki Kaisha Bio Arts), 19 April 2007 (19.04.2007), entire text; all drawings (Family: none) | 1-19 |
| A | WO 2008/096813 A1  (Hitachi Medical Corp.), 14 August 2008 (14.08.2008), entire text; all drawings & US 2010/0104159 A1    & CN 101605498 A | 1-19 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 4387644 B